# EUROPEAN PATENT SPECIFICATION

(11) **EP 3 880 218 B1**
(45) Date of publication and mention of the grant of the patent: **14.05.2025**
(21) Application number: 19809176.1
(22) Date of filing: 15.11.2019
(51) Int. Cl.: A61K 35/644, A61K 31/164, A61K 31/198, A23L 33/10, A23L 21/20, A61K 9/46, A61K 9/00, A61K 9/20, A61P 37/04

(54) **FOOD SUPPLEMENTS COMPRISING PROPOLIS**
PROPOLIS ENTHALTENDE NAHRUNGSERGÄNZUNGSMITTEL
COMPLÉMENTS ALIMENTAIRES COMPORTANT DE LA PROPOLIS

(30) Priority: 16.11.2018 EP 18206876
(43) Date of publication of application: 22.09.2021
(73) Proprietor: Specchiasol S.r.l., 37012 Bussolengo (VR) (IT)
(72) Inventor: SCIALPI, Antonio I, 37012 Bussolengo (Verona) (IT)
(74) Representative: Petraz, Gilberto Luigi
(86) International application number: PCT/IB2019/059826
(87) International publication number: WO 2020/100098

(56) References cited:
- IT-A1- BO20 090 487
- COHEN HERMAN A ET AL: "Effectiveness of an herbal preparation containing echinacea, propolis, and vitamin C in preventing respiratory tract infections in children. A randomized, double-blind, placebo-controlled, multicenter study", ARCHIVES OF PEDIATRICS AND ADOLESCENT MEDICINE, AMERICAN MEDICAL ASSOCIATION, CHICAGO, IL, US, vol. 158, no. 3, 1 March 2004 (2004-03-01), pages 217 - 221, XP002594097, ISSN: 1072-4710
- J. M. KEPPEL HESSELINK ET AL: "Palmitoylethanolamide: A Natural Body-Own Anti-Inflammatory Agent, Effective and Safe against Influenza and Common Cold", INTERNATIONAL JOURNAL OF INFLAMMATION, vol. 5, no. 1, 1 January 2013 (2013-01-01), pages 437 - 8, XP055197977, ISSN: 2090-8040, DOI: 10.1155/2013/151028
- NAKAJIMA YOSHIMI ET AL: "Comparison of bee products based on assays of antioxidant capacities", BMC COMPLEMENTARY AND ALTERNATIVE MEDICINE, BIOMED CENTRAL LTD., LONDON, GB, vol. 9, no. 1, 26 February 2009 (2009-02-26), pages 4, XP021049633, ISSN: 1472-6882, DOI: 10.1186/1472-6882-9-4

## Description

### TECHNICAL FIELD

The present invention refers to the combination of food grade substances for making benefits in the states of influenza and common colds.

The present invention also refers to method for producing food supplements, functional foods, foods for special medical purposes or medicaments comprising the aforesaid combinations and allowing to maintain the physiological state of health of a user against seasonal illnesses such as influenza and common colds. The present invention is preferably and advantageously applied for buccal, peribuccal, orobuccal or sublingual administration in the form of orosoluble and/ or effervescent powder, tablets, capsules, bars, dragees, pills or granules.

### PRIOR ART

Several food supplements and the like intended to prevent and/or treat seasonal illnesses such as influenza and common colds are known so far.

In particular, combinations of Propolis, which is a natural antibiotic, and natural anti inflammatory substances, such as Echinacea and similar plants, are known so far. Studies conducted using Propolis in combination with natural extracts such as Echinacea are reported in the scientific literature.

On the contrary, to the Applicant's knowledge, no studies were conducted to evaluate the effectiveness, for example, of the Devil's claw and Propolis combination; Devil's claw contains iridoid glycosides, a class of compounds that has demonstrated anti inflammatory properties.

An example of this combination is reported in Arch. Pediatr. Adolesc. Med. 2004; 158(3): 217-2. Effectiveness of an herbal preparation containing Echinacea, Propolis, and vitamin C in preventing respiratory tract infections in children: a randomized, double-blind, placebo-controlled, multicenter study. This study demonstrates that an herbal preparation containing an extract of Echinacea, Propolis, and vitamin C has a significant beneficial effect on the incidence and severity of respiratory tract infections in young children.

The evidence on orally administered Echinacea extracts for acute upper respiratory tract infections concluded that they might be beneficial for the early treatment of existing illness but not for prevention.

Moreover, the safety data on Echinacea are relatively strong.

Low frequency of adverse effects, such as unpleasant taste, nausea or vomiting, abdominal pain, and diarrhea was observed in the study.

One limitation of the study is the lack of appropriate quality control and standardization, because the active components of the preparations are not known.

In addition, the safety of long-term prophylactic use was not tested.

This study suggests that an herbal extract preparation containing Echinacea, Propolis, and vitamin C is beneficial for the prevention of respiratory tract infections in children. However, considering that this is the first trial conducted in young children, conclusions must be made with caution. Additional studies are needed in larger samples to confirm the findings and to rule out potential adverse effects in general or specific populations at risk, such as allergic children or those receiving co -therapy or having different morbidities, before the preparation can be recommended for routine clinical use.

Vitamin C can also be used in addition to the aforesaid substances for its immuno- shmulant action.

An example of this combination is described in RU2246961C2: Preparation "Propovit with vitamin C" eliciting anti-inflammatory and antibacterial properties.

However, the drawbacks of this solution mainly reside in the fact that the absence of N-Acetyl Cysteine (NAC) and PalmitoylEthanolAmide (PEA) precludes the mucolytic and inflammatory activity, respectively. Furthermore, the Propolis extract used does not appear to be purified and this significantly lowers its antibacterial activity. Overall, the preparation reported in RU2246961 exerts only antibacterial action, due to the presence of Propolis, and antioxidant due to ascorbic acid.

Moreover, a combination of vitamin C and Propolis was tested in the treatment of diabetic wounds, as reported in Voss et al. Int. J. Pharm. 2018;552(1-2):340-351. Polysaccharide-based film loaded with vitamin C and Propolis: A promising device to accelerate diabetic wound healing.

However, the drawbacks of this solution mainly reside in the fact that the formulation was for external use and it has been tested as antibacterial and healing. Mucolytic and anti-inflammatory activity not evaluated.

Moreover, a commercial product containing Propolis and Vitamin C, PropolNAC<^{®}>, is available in microencapsulated powder.

However, this formulation is not very effective in flu and cold diseases because it lacks the mucolytic action and the anti-inflammatory action is bland.

Also combinations of Propolis and N-Acetyl Cysteine (NAC) are known so far. For example, a combination of Propolis and NAC has been tested for both antioxidant and anti-inflammatory potential and it is reported in Zujovic et al.

(Testing the antioxidant and anti-inflammatory potential of Propolis and N-Acetylcysteine combination. Conference: American Thoracic Society 2019 International Conference, May 17-22, 2019 - Dallas, TX. DOI: 0.1164/ ajrccm-conference.2019.199.1_MeetingAbstracts.A2196).

The aforesaid preparation containing NAC and Propolis in the treatment of the acute respiratory infection, during the course of ten days, showed a positive effect via stabilizing the overall condition, inducing coughing of the viscous secretions.

Moreover, a combination of NAC and Propolis was reported in Videnovic-Ivanov J. et al. "Evaluation of Efficacy and Tolerability of a Fixed Combination of N-Acetyl Cysteine (NAC) with Propolis as a Supplement in Treatment of Adults with Acute Respiratory Infections a Real Life Observational Single Centre Clinical Trial". EC Pulmonology and Respiratory Medicine 8.5 (2019): 417-423.

Moreover, a commercial product containing Propolis and NAC, PropolMucil<^{®}>, is available.

The preparations containing Propolis and NAC have antibacterial activity due to Propolis and mucolytic activity due to NAC, but the main drawback of these solutions resides in that the anti-inflammatory activity is almost completely absent.

In particular, the several solutions that have been proposed so far for preventing and/ or treating seasonal illnesses such as influenza and common colds include:
- Propolis;
- natural anti-inflammatory substances, such as the devil's claw and similar plants;
- vitamin C; and
- N- Acetyl Cysteine (NAC).

Nevertheless, due to the above-mentioned drawbacks, the combinations available so far can not be effectively used.

Therefore, there still exists the need of suitable food supplements, functional foods, foods for special medical purposes or medicaments for maintaining the physiological state of health of a user against seasonal illnesses, such as influenza and/ or common colds, that can be effectively used, besides being inexpensive, simply usable and easily produced.

In brief, up to the present time, to the Applicant's knowledge, there are no known solutions allowing to provide combinations of food grade substances and the methods for producing such combinations so that:
- the broad spectrum and antimicrobial functions of a natural antibiotic with the analgesic and anti-inflammatory function of a natural and endogenous substance can be combined;
- the antioxidant, anti-radical and mucolytic activity of a nutritional supplement with the analgesic and anti-inflammatory function of a natural and endogenous substance can be exploited;

an effectiveness higher than that of the current commercial products for influenza and/ or common colds can be achieved; and
a low-cost and simple production method can be realized.

Therefore, the Applicant, with the combinations of food grade substances and the methods for producing such combinations according to the present invention, intends to remedy such lack.

### OBJECTS AND SUMMARY OF THE INVENTION

It is an object of the present invention to overcome the drawbacks of the known prior art related to food supplements, functional foods, foods for special medical purposes or medicaments for maintaining the physiological state of health of a user against seasonal illnesses, such as influenza and/or common colds.

The present invention intends to solve the problem of providing anti-flu and anti inflammatory products.

Specifically, the present invention intends to solve the problem of enhancing the effectiveness of the aforesaid products as far as anti-flu and anti-inflammatory properties thereof are concerned.

In particular, the present invention aims at combining the broad spectrum and antimicrobial functions of a natural antibiotic with the analgesic and anti inflammatory function of a natural and endogenous substance.

The present invention also aims at exploiting the antioxidant, anti-radical and mucolytic activity of a nutritional supplement with the analgesic and anti inflammatory function of a natural and endogenous substance.

The present invention also aims at using, in some formulations for the treatment of cold or influenza, the Devil' Claw extract in association with Propolis and NAC.

The present invention also aims at providing low-cost and simple production methods.

The above-stated problems are solved by the present invention, which provides combinations allowing
- to combine the broad spectrum and immuno -stimulant antimicrobial function of Propolis with the anti-inflammatory function of PalmitoylEthanolAmide (PEA), and
- to exploit the antioxidant, anti-radical and mucolytic function of N-Acetyl Cysteine (NAC) with the anti-inflammatory function of PalmitoylEthanolAmide (PEA), as well as various mixtures and recipes of such substances.

These combinations will be used in food supplements, functional foods, foods for special medical purposes or medicaments to maintain the physiological state of health of a user against typical seasonal illnesses such as influenza and cold. The aforesaid and other objects and advantages of the invention, as will appear evident from the following description, are achieved with combinations of food grade substances according to claim 1 or 2. Moreover, the aforesaid and other objects and advantages of the invention are achieved with production methods according to claim 9 or 10.

Preferred embodiments and variants of the combinations and of the methods of the present invention are the subject-matter of the dependent claims.

It is understood that all the annexed claims form an integral part of the present description and that each of the technical features therein claimed is possibly independent and autonomously usable with respect to the other aspects of the invention.

It will be immediately evident that several modifications (for example relevant to shape, sizes, arrangements and parts with equivalent functionality) could be brought to what described without departing from the scope of the invention as claimed in the appended claims.

Advantageously, the technical solution according to the present invention allows to: achieve an effectiveness higher than that of the current commercial products for influenza and/ or common colds;
- be inexpensive;
- be simply usable; and
- realize low-cost and easy production methods.

Further advantageous features will appear more evident from the following description of preferred but not exclusive embodiments, merely given by way of explanatory and not limiting example.

### BRIEF DESCRIPTION OF THE DRAWINGS

The present invention will be described hereinbelow by means of some preferred embodiments, given by way of explanatory and not limiting example, with reference to the accompanying drawings. These drawings illustrate different aspects and examples of the present invention and, where appropriate, similar structures, components, materials and/or elements in different figures are denoted by similar reference numbers.
Figure 1 shows an overview of the antioxidant action of NAC (see Aldini et. al. 2018); Figure 2 shows the mucolytic action of the NAC; activity is due to its ability to break the disulphide bridges of the high-molecular-weight glycoproteins in the mucus, resulting in reduced viscosity;
Figure 3 shows the plasma levels of TNFa in control (A, C1-C6) and treated mice (B, T1-T6); control mice received HFD while treated HFD containing PEA, NAC, purified Propolis extract and ascorbic acid;
Figure 4 shows the plasma levels of IL-6 in control (A, C1-C6) and treated mice (B, TI TO); control mice received HFD while treated HFD containing PEA, NAC, purified Propolis extract and ascorbic acid;
Figure 5 shows the process flow, included CCP, for manufacturing of effervescent tablets;
Figure 6 is a flow chart showing the steps of a first embodiment of the method according to the present invention;
Figure 7 is a flow chart showing the steps of a second embodiment of the method according to the present invention; and
Figure 8 reports the main commercial products that may present similarities with the applicant's formulations. It can be seen that the composition and the "medical claims" of the products already present on the market is not comparable or superimposable to that shown in the present invention.

### DETAILED DESCRIPTION OF THE INVENTION

While the invention is susceptible of various modifications and alternative constructions, some preferred embodiments are shown in the drawings and will be described in detail hereinbelow.

It should be understood, however, that there is no intention to limit the invention to the specific illustrated embodiments but, on the contrary, the invention intends to cover all the modifications, alternative constructions and equivalents that fall within the scope of the invention as defined in the claims.

In the following description, therefore, the use of "for example", "etcetera" and "or" denotes non-exclusive alternatives without limitation, unless otherwise indicated; the use of "also" means "among, but not limited to", unless otherwise indicated; the use of "includes / comprises" means "includes / comprises, but not limited to", unless otherwise indicated.

In the present specification, the following terms have the following meanings:
"combination(s)" means mixture(s) obtained when two or more substances are combined and/ or an arrangement of two or more substances in a particular order;
"food grade substance(s)" means material(s) non-toxic and safe for consumption;
"association(s)" means aggregation(s) of chemical species and/ or natural extracts to form loosely bound complexes;
"purified" means free from undesirable elements;
"food supplement(s)" are concentrated sources of nutrients or other substances with a nutritional or physiological effect that are marketed in "dose" form (e.g. pills, tablets, capsules, liquids in measured doses); a wide range of nutrients and other ingredients might be present in food supplements, including, but not limited to, vitamins, minerals, amino acids, essential fatty acids, fiber and various plants and herbal extracts; food supplements are intended to correct nutritional deficiencies, maintain an adequate intake of certain nutrients, or to support specific physiological functions; they are not medicinal products and, as such, they cannot exert a pharmacological, immunological or metabolic action; therefore, their use is not intended to treat or prevent diseases in humans or to modify physiological functions; in the European Union, food supplements are regulated as foods; a harmonized legislation regulates the vitamins and minerals, and the substances used as their sources, which can be used in the manufacturing of food supplements; for ingredients other than vitamins and minerals, the European Commission has established harmonized rules to protect consumers against potential health risks and maintains a list of substances which are known or suspected to have adverse effects on health and the use of which is therefore controlled (European Food Safety Authority, EFSA);
"functional food(s)" means dietary items that, besides providing nutrients and energy, beneficially modulate one or more targeted functions in the body, by enhancing a certain physiological response and/ or by reducing the risk of disease (Food Structures, Digestion and Health, 2014);
"food(s) for special medical purposes", FSMP(s), are designed to feed patients who, because of a particular disease, disorder or medical condition, have nutritional needs that cannot be met by consuming standard foodstuffs; specifically, according to European Union legislation, they are intended for patients with a limited, impaired or disturbed capacity to take, digest, absorb, metabolize or excrete ordinary foods, or certain nutrients or metabolites; or with other medically nutrient requirements whose dietary management cannot be achieved by modification of the normal diet alone; FSMP(s) should be used only under medical supervision and must carry labelling information about their intended use (European Food Safety Authority, EFSA);
"medicament(s)" means substance(s) used in therapy;
"physiological state of health of a user" means the condition or the state of the body or bodily functions.

The present invention is based on the innovative concept of providing combinations and production methods by exploiting the anti-inflammatory action of PalmitoylEthanolAmide (PEA).

Specifically, the present invention provides more effective products to be used for preventing and/ or treating influenza and cold.

PalmitoylEthanolAmide (PEA) acts with a different mechanism from the other known anti-inflammatory substances and, in particular, it operates on the mastocytes.

PalmitoylEthanolAmide (PEA) is a non-endocannabinoid lipid mediator belonging to the class of the N-acylethanolamine, which includes N-arachidonoyl-ethanolamine (anandamide; AEA) and A-oleoyl-ethanol amine (OEA), an anorectic mediator.

The chemical structure of PalmitoylEthanolAmide is as follow:
Image available on "Original document"

Either preclinical or clinical studies indicate that PEA could be useful in a wide range of therapeutic areas, including eczema, pain and neurodegeneration. Depending on the country, several PEA-based products such as Normast<^{®}>, Mastocol<^{®}>, Nevamast<^{®}>and etcetera are already licensed for use in humans. Thus, PEA is found in nutraceutical, a food supplement, or a food for medical purposes.

PEA is generally used in humans for its analgesic and anti-inflammatory properties (see Tsuboi et al., Endocannabinoids and related N-acylethanolamines: biological activihes and metabolism. Inflamm. Regen. 2018, 38, 28) and has demonstrated high safety and tolerability (see Gabrielsson et al., Palmitoylethanolamide for the treatment of pain: pharmacokinetics, safety and efficacy. Br. J. Clin. Pharmacol. 2016, 82, 932- 942).

Regarding the action mechanism of PalmitoylEthanolAmide (PEA), preclinical studies demonstrated that PEA induced its biological effects by acting on several molecular targets (see Tsuboi et al., Endocannabinoids and related N-acylethanolamines: biological activities and metabolism. Inflamm. Regen. 2018, 38, 28).

At first, it was thought that PEA exercised its anti-inflammatory effects through the down regulation of the mast cells.

The most recent research seem supported the view that PEA directly activates certain specific receptors such as PPAR-a and GPR55; this is particularly important because PPAR-a is known for its protective role against inflammation, and consequently PPAR-a ligands are recognized as possible anti-inflammatory compounds.

In this regard, it has been shown in vitro that PEA was able to activate PPAR-a with an EC50 of approximately 3.1 mM (see De Gregorio et al., Role of palmitoylethanolamide (PEA) in depression: translational evidence. J. Affect. Disord. 2018, 255, 195-200).

Recently, Lowin et al. (Anti-inflammatory effects of N-acylethanolamines in rheumatoid arthritis synovial cells are mediated by TRPV1 and TRPA1 in a COX -2 dependent manner. Arthritis Res. Ther. 2015, 17, 321) studied the influenza of anandamide (AEA), PalmitoylEthanolAmine (PEA) and oleylethanolamine (OEA) on several features of arthritic inflammation in vitro and in vivo ; the authors reported that N-acylethanolamines exert anti-inflammatory effects in synovial fibroblasts.

This study demonstrates that cytokine production of synoviocytes was modulated by the N-acylethanolamines.

The effects of these compounds were enhanced by COX-2 inhibition suggesting a sensitizing role for COX-2-derived metabolites at Transient Receptor Potential Ankyrin (TRPs).

These findings show that activation of the endocannabinoid system can be beneficial in arthritis and manipulation of this system (especially by combination of a COX -2 and a Fatty Acid Amide Hydrolase inhibitor) might be a promising strategy to reduce erosions and inflammation in arthritis.

Without wishing to be bound by this theory, the Applicant deems that PalmitoylEthanolAmide (PEA) interacts with Propolis and N-Acetyl Cysteine (NAC) in such a way as to make the action of prevention and treatment of flu and cold more efficient and effective.

PEA compared to other natural anti-inflammatories such as the Devil's claw has a vast collection of literature proving its effectiveness as anti-inflammatory both at the peripheral level and in the Central Nervous System (CNS).

Moreover, recently the mechanisms of action by which the PEA exercises its biological activities have been clarified.

Moreover, it should be emphasized that PEA is an endogenous compound considered an autacoid, i.e. a physiologically active substance produced by the body.

Indeed, PEA can be produced in almost every cell by on-demand synthesis when cells or tissues are damaged, or are threatened to become damaged. PalmitoylEthanolAmide restores unbalanced regulatory biological processes that may be disturbed, acutely or chronically, by a host of endogenous and exogenous mechanisms.

Independent aspects of the present invention, which will be described in detail hereinafter, refer to:
- combinations of food grade substances; and
- methods to produce food supplements, functional foods, foods for special medical purposes or medicaments for maintaining the physiological state of health of a user against seasonal illnesses.

According to a first embodiment of the invention, a combination of food grade substances comprises: - a first association comprising purified Propolis and PalmitoylEthanolAmide (PEA), and
- a second association comprising PalmitoylEthanolAmide (PEA) and N-Acetyl Cysteine (NAC).

According to a second embodiment of the invention, a combination of food grade substances comprises:
- purified Propolis,
- PalmitoylEthanolAmide (PEA), and
- N- Acetyl Cysteine (NAC).

The above-mentioned combinations are intended for use as food supplements, functional foods, foods for special medical purposes or medicaments for maintaining the physiological state of health of a user against seasonal illnesses. Preferably, the seasonal illnesses are influenza and/ or common colds.

The above-mentioned combinations are formulated for buccal, peribuccal, orobuccal or sublingual administration.

Preferably, the combinations are in the form of orosoluble and/or effervescent powder, tablets, capsules, bars, dragees, pills or granules.

According to the first embodiment of the invention, preferably purified Propofis and PalmitoylEthanolAmide (PEA) of the first association are in a ratio by weight ranging between 0.2 and 5 and PalmitoylEthanolAmide (PEA) and N-Acetyl Cysteine (NAC) of the second association are in a ratio by weight ranging between 0.2 and 4.

According to the second embodiment of the invention, preferably purified Propolis and PalmitoylEthanolAmide (PEA) and N-Acetyl Cysteine (NAC) are in a ratio by weight ranging between 0.1 and 6.

The properties of the individual components of the present invention are reported hereinbelow.

### PROPOLIS

Propolis appropriately purified and standardized thanks to the polyphenol content exerts a broad spectrum antimicrobial activity against various microbial species such as bacteria, fungi, molds and viruses (see Sforcin et al., Biological Properties and Therapeutic Applications of Propolis. Phytother. Res. 2016, 30, 894-905).

Recent studies have also shown that Propolis can be useful for the immune system activity of adults and children. The activity of Propolis depends on both low impurity content and a high polyphenol content, the latter determined by HPLC coupled to diode array detector and/ or mass spectrometer.

The correct composition and method for the determination in functional polyphenols of Propolis such as flavonoids and phenolic acids, which guarantees the desired effects according to the present invention, is reported in the study of Gardana et al. (see Analysis of the polyphenolic fraction of Propolis from different sources by liquid chromatography-tandem mass spectrometry is carried out in accordance with the study. J. Pharm. Biomed. Anal. 2007, 45, 390-9).

To underline that the results of the study evidence the presence of several flavonoids in human plasma after ingestion of a purified and dewaxed Propolis extract (EPID). This indicates that polyphenols from this extract are absorbed, metabolized and, therefore, may exert systemic effects, such as protection of lipid membranes from oxidative injury and immuno-shmulation (see Gardana et al., Evaluation of propolis polyphenols absorption in humans by liquid chromatography/tandem mass spectrometry. Rapid Commun. Mass Spectrom. 2007. 21, 3849-3854).

### PALMITOIL ETHANOLAMIDE (PEA)

PalmitoylEthanolAmide, or PEA, is the amide of a fatty acid of an endogenous nature and belongs to the class of nuclear factor agonists. PEA performs a wide variety of biological functions related to chronic pain and inflammation; it is thought that the main target is PPAR-a. In the current state of knowledge, PEA is considered a food supplement with anti-inflammatory and pain-relieving properties.

PEA acts on mediators that support inflammatory processes and it is able to restore the correct functioning of both tissues and various organs; this substance, in fact, is active orally during the lowering of inflammation linked to the tissue. Moreover, it is active during mast cell degranulation in vivo reducing hyperalgesia due to compression of the peripheral nerve.

PEA is an active ingredient that has many potentials, not exclusively related to inflammation. Indeed, it is also related to the treatment of chronic pain, as well as multiple sclerosis and various autoimmune diseases (see Keppel et al., Palmitoylethanolamide: A Natural Body-Own Anti-Inflammatory Agent, Effective and Safe against Influenza and Common Cold. Int. J. Inflamm. 2013, 2013, 151028).

### N- Acetyl Cysteine (NAC)

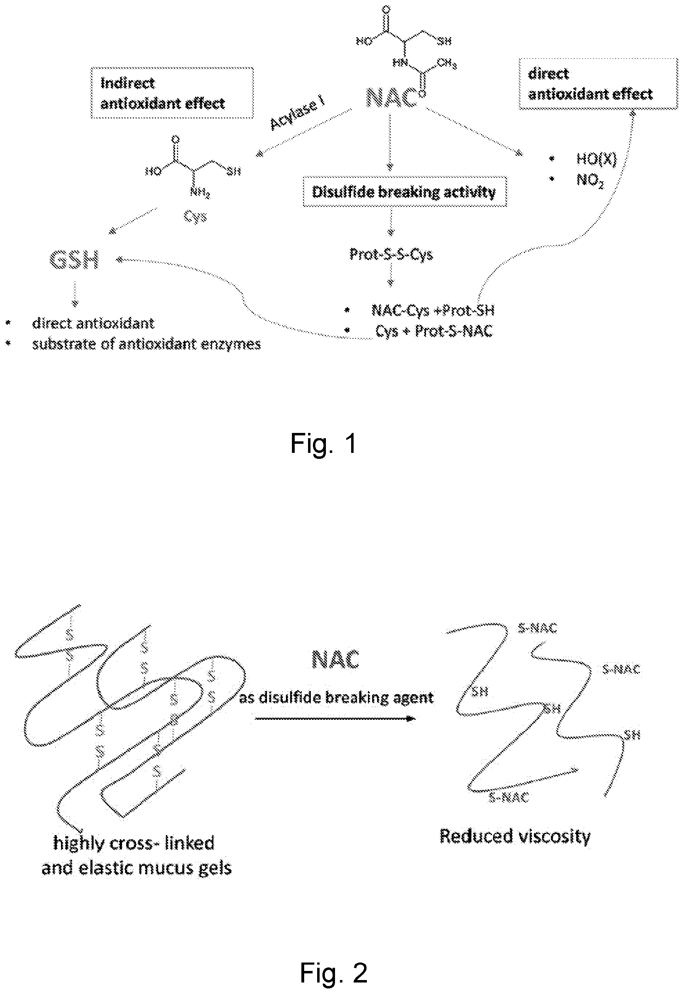

NAC is a molecule derived from the acetylation of the amino acid cysteine widely used in the health treatment market due to its dual activity:
- firstly as antioxidant and anti-radical: the antioxidant effect is due to the ability of NAC to act as a reduced glutathione (GSH) precursor; GSH is a well-known direct antioxidant and a substrate of several antioxidant enzymes; moreover, in some conditions where a significant depletion of endogenous Cys and GSH occurs, NAC can act as a direct antioxidant for some oxidant species such as NO2 and HOX; the antioxidant activity of NAC could also be due to its effect in breaking thiolated proteins, thus releasing free thiols as well as reduced proteins, which in some cases, such as for mercaptoalbumin, have important direct antioxidant activity (see Aldini et al. N- Acetylcysteine as an antioxidant and disulphide breaking agent: the reasons why. Free Radical Res., 2018, 52, 751-762); an overview of the antioxidant action of NAC (see Aldini et. al. 2018) is shown in Fig. 1;
- secondly as mucolytic: NAC has a specific action in fluidifying mucus; when the mucous secretions are fluid the process of expelling the secretions is easier and it is easier for the body to get rid of the infectious agents; the disulphide breaking activity of NAC explains its mucolytic activity that is due to its effect in reducing heavily cross- linked mucus glycoproteins (see Aldini et al. N-Acetylcysteine as an antioxidant and disulphide breaking agent: the reasons why. Free Radical Res. 2018, 52, 751-762.

As far as the mucolytic action of NAC is concerned, its activity is due to NAC ability to break the disulphide bridges of the high-molecular-weight glycoproteins in the mucus, resulting in reduced viscosity (see Aldini et. al. 2018) ) as shown in Fig. 2. NAC is also used to promote the action of antimicrobial substances against the infectious agents that form Biofilm.

The combinations according to the present invention are described in detail below with reference to the following Examples, which have been developed, based on experimental data and which are intended to be illustrative but not limitative of the present invention.

### Example 1 - Effervescent tablets for oral use

Palmitoylethanolamide (PEA) 100 mg
N-acetylcysteine (NAC) 300 mg
Dewaxed and Purified Propolis 100 mg
(Intake of polyphenols) (3 mg)
Rose hip fruit extract 286 mg
(intake in vitamin C) (200 mg)
Devil's claw root d.e. 25 mg
Citric acid 2300 mg
Sodium bicarbonate 1240 mg
Sweeteners 30 mg
Flavouring 110 mg
Vitamin group B lmg

### Example 2 - Tablets for oral use

Palmitoylethanolamide (PEA) 600 mg
N-acetylcysteine (NAC) 500 mg
Binding agents 1600 mg
Sweeteners 90 mg
Flavouring 700 mg
Vitamins group B 10 mg

### Example 3 - Tablets for oral use

Dewaxed and Purified Propolis 200 mg
(Intake of polyphenols) (6 mg)
N-acetylcysteine (NAC) 200 mg
Balsam 140 mg
Grindelia extract 140 mg
Honey 160 mg
Vitamin C 40 mg Binding agents 3790 mg
Sweeteners 60 mg
Flavouring 230 mg

### Example 4 - Tablets for sublingual use

Palmitoylethanolamide (PEA) 330 mg
N-acetylcysteine (NAC) 370 mg
Rose hip fruit 90 mg
(intake in vitamin C) (63 mg)
Binding agents 120 mg
Sweeteners 6 mg
Flavouring 20 mg
Vitamin B 1 mg

### Example 5 - Powder for oral use (Powder in to the cap of the bottle, lOmL)

Dewaxed and Purified Propolis 30 mg
Palmitoylethanolamide (PEA) 120 mg
N-acetylcysteine (NAC) 120 mg
Rose hip fruit 30 mg
(intake in vitamin C) (21 mg)
Binding agents 10 mg
Sweeteners 2 mg
Flavouring 7 mg
Vitamin B 5 mg

### Example 6 - Cough syrup

Dewaxed and Purified Propolis 300 mg
N-acetylcysteine (NAC) 300 mg
Palmitoylethanolamide (PEA) 100 mg
Honey 500 mg
Acerola 250 mg
(intake in vitamin C) (62.5 mg)
Grindelia e.s. 10 mg
Sweeteners 2 mg Emulsifier 18 mg
Flavouring 5 mg
Thickening agents 15 mg

The combinations according to the present invention have been tested as described below.

The purpose of the study was to assess the effect of a dietary supplement containing PEA, NAC, ascorbic acid (AA) and purified extract of Propolis on the inflammatory state in mice fed a high-fat diet (HFD). Inflammation was induced in male C57/B16 mice by feeding a HFD. Dietary supplement was provided instead of food. Body weight and food intake were measured regularly over a 2-week period. Serum levels of the pro-inflammatory cytokines TNFa and IL-6 were investigated. Dietary supplementation of a Formulation containing PEA, NAC, AA and purified extract of Propolis significantly reduced plasma levels of the cytokines TNFa and IF-6.

### Materials and methods

### Mice

Two months old wild-type mice (C57/B16 strains, Charles River) were housed individually in an air-conditioned room (T 20±5 °C, RH 60±10%). Water was available ad libitum to the mice and the vessel containing the food replaced every day, liquid and solid food consumption controlled. Duration of all treatment was 2 weeks. Blood was collected, centrifuged and serum stored at -80 °C. All the experiments with mice were performed in accordance with the Italian and EU Taws.

### Diet

HFD for Diet-Induced Obesity D12492 was provided by Research Diets, Inc., New Brunswick, NJ, USA). The energy provided to the mice was approximately 5.0 kcal/ g. The control mice (n=6, C1-C6) received 10 g of diet per day while 100 mg of a formulation containing PEA, NAC and Propolis extract was added to the diet of the test mice (n=6, T1-T6). The tested formulation contained PEA (2.2 mg), NAC (6.7 mg), dewaxed and purified Propolis extract (2.2 mg), Rose hip fruit extract (6.4 mg), Devil's claw root extract (0.6 mg), citric acid (51.2 mg), sodium bicarbonate (27.6 mg), sweeteners (0.7 mg), flavorings (2.4 mg) and riboflavin (0.02 mg). Measurements of cytokines

Plasma cytokine TNFa and IL-6 were analyzed using a commercially available ELISA kits (Thermo Fisher Scientific), following the manufacturer's instructions.

### Results

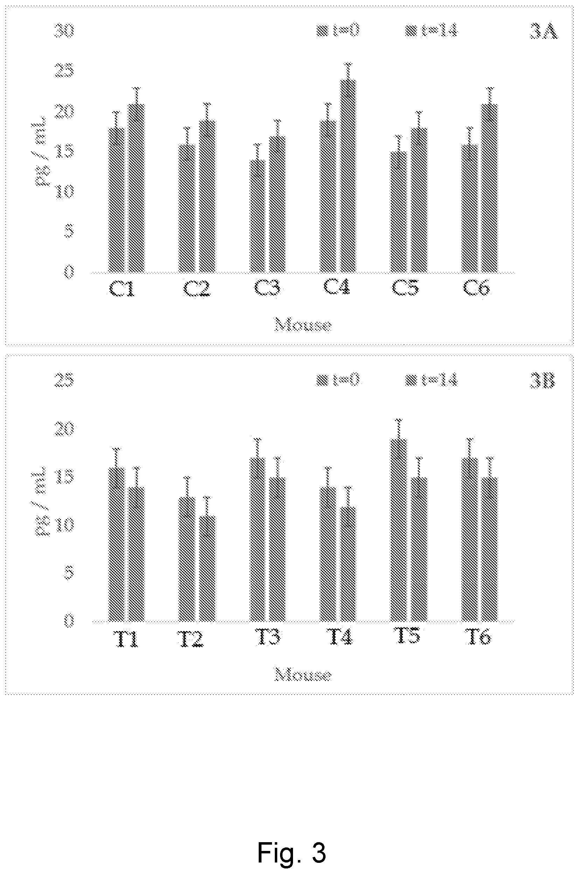

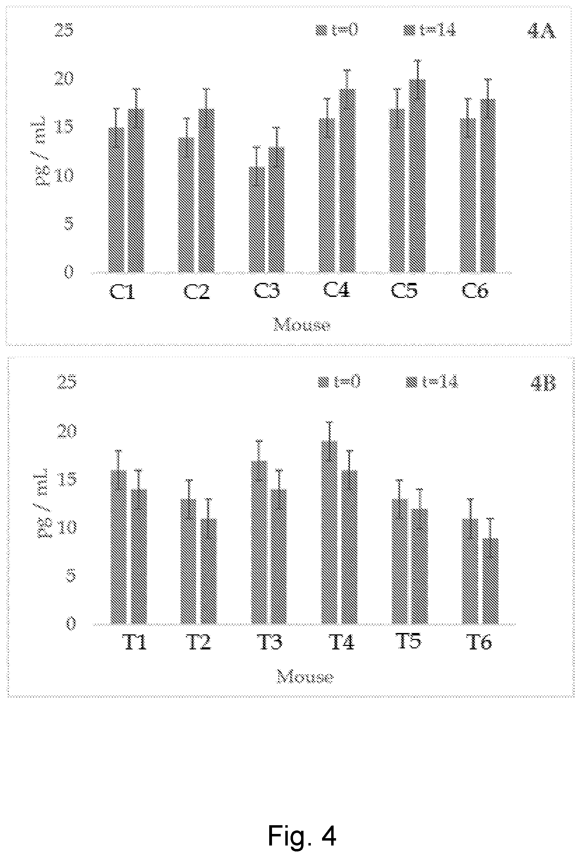

No significant difference in weight between the two groups, control and treated, was detected after 2 weeks treatment. Mice fed HFD showed a significant increase in TNFa (p<0.01) and IL-6 (p<0.01) plasma levels compared to those treated with HFD- formulation. Figure 3 and 4 shows the plasma levels of TNFa and IL-6, respectively, in control mice (A, C1-C6) and treated (B, T1-T6) with the formulation containing PEA, NAC, purified Propolis extract and vitamin C. Post doc analysis showed that TNFa ( p < 0.001) and IL-6 (p < 0.01) at plasma level were downregulated by formulation treatment (Figure 3 and 4). Regarding TNFa, in control and treated mice a variation of 22±4% and -14±3% was established after 2 week of treatment, respectively. For the IL- 6, 17±3% and - 15±4% change in control and treated mice, respectively.

In the mice, the tested Formulation was able to reduce cytokine production, decreasing the inflammatory state. Therefore, it is conceivable to hypothesize that administration of the Formulation could directly impact inflammation. With reference to FIG. 3 it is observed that the graph shows the plasma levels of TNFa in control (3A, C1-C6) and treated mice (3B, T1-T6). Control mice received HFD while treated HFD containing PEA, NAC, purified Propolis extract and ascorbic acid.

With reference to FIG. 4 it is observed that the graph shows the plasma levels of IL-6 in control (4A, C1-C6) and treated mice (4B, T 1-T6). Control mice received HFD while treated HFD containing PEA, NAC, purified Propolis extract and ascorbic acid.

The combinations according to the present invention have been compared with known solutions, as described below.

A market research has shown that two products PEA -based, both also contain acetyl- carnitine, are commercially available: the first one, Lipease LAC, is a food for special medical purposes containing for the treatment of pain; the second one, Nervax PEA, helps to reduce tiredness and fatigue. A third commercial product, Lipease forte, contains PEA and lipoic acid as active ingredients. It is a dietary supplement useful in patients with disorders sustained by neuro-inflammatory processes.

To the Applicant's knowledge there are no commercial preparations containing Propolis, NAC and PEA for the treatment of influenza and cold.

In particular, the formulation of the present invention mixes the antibacterial action of the Propolis, mucolytic of the NAC and anti-inflammatory of the PEA. Regarding the latter component, PEA, a study was conducted to evaluate the anti inflammatory activity and the results obtained confirmed the biological action of this compound.

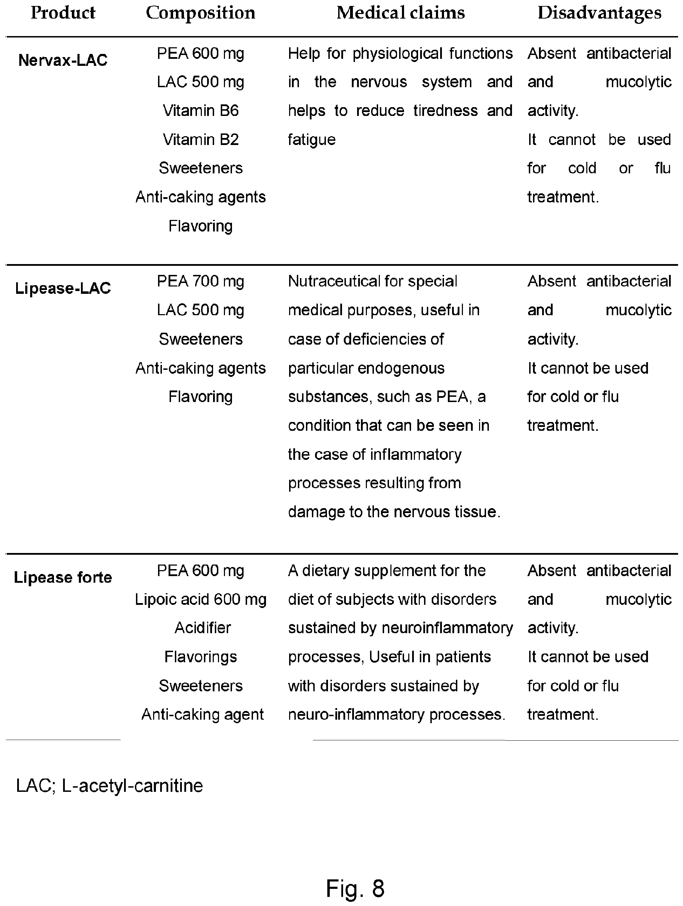

The results of the comparison between the present invention and the known solutions are summarized in the table shown in FIG. 8.

The comparative table shows the main commercial products that may present similarities with the applicant's formulations. From the data reported in the table it can be seen that the composition and the "medical claims" of the products already present on the market is not comparable or superimposable to that shown in the present invention. Indeed, the present invention refers to the combination of food grade substances for making benefits in the states of influenza and common colds. On the contrary, the formulations reported in the table have as "medical claim" the treatment of inflammatory states, above all on the nervous system.

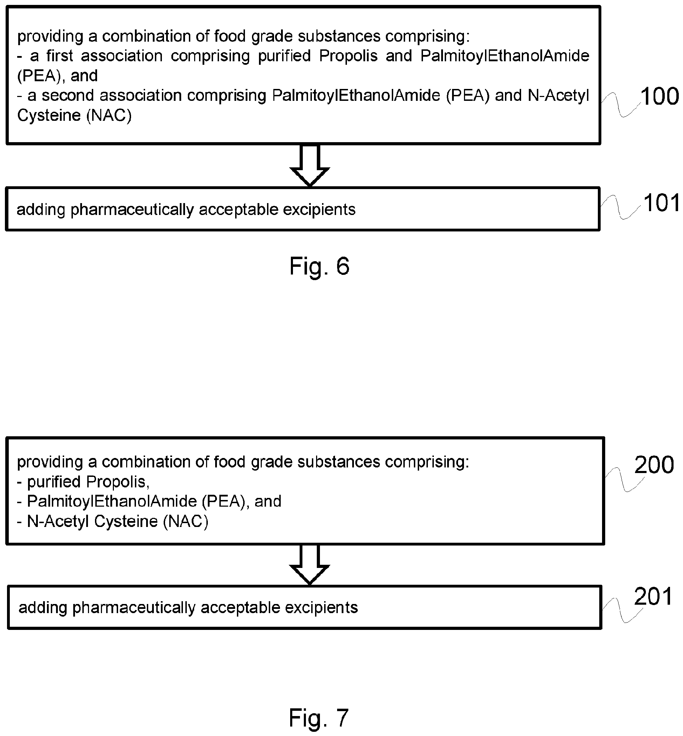

With reference to FIG. 6, a method to produce food supplements, functional foods, foods for special medical purposes or medicaments for maintaining the physiological state of health of a user against seasonal illnesses comprises the steps of
- providing the combination of food grade substances comprising a first association comprising purified Propolis and PalmitoylEthanol Amide (PEA), and a second association comprising PalmitoylEthanolAmide (PEA) and N-Acetyl Cysteine (NAC) (step 100),
- adding pharmaceutically acceptable excipients (step 101).

With reference to FIG. 7, a method to produce food supplements, functional foods, foods for special medical purposes or medicaments for maintaining the physiological state of health of a user against seasonal illnesses comprises the steps of
- providing the combination of food grade substances comprising purified Propolis, PalmitoylEthanol Amide (PEA) and N- Acetyl Cysteine (NAC) (step 200),
- adding pharmaceutically acceptable excipients (step 201).

Examples of pharmaceutically acceptable excipients that can be used in the present invention are:
- cellulose, magnesium stearate, sodium bicarbonate, sodium silicate, silicon dioxide, talcum powder, bentonite, stearic acid, polyvinylpirrolidone, polysorbates and polydimethylsiloxane;
- steviolgly co sides, erythritol, maltodextrin, sucralose, neohesperidin, polyols (xylitol, mannitol, sorbitol, etcetera), sugar (fructose, glucose, sucrose);
- citric acid, lactic acid, tartaric acid;
aroma compound (vanilla, strawberry, lemon, etcetera);
- essential oil.

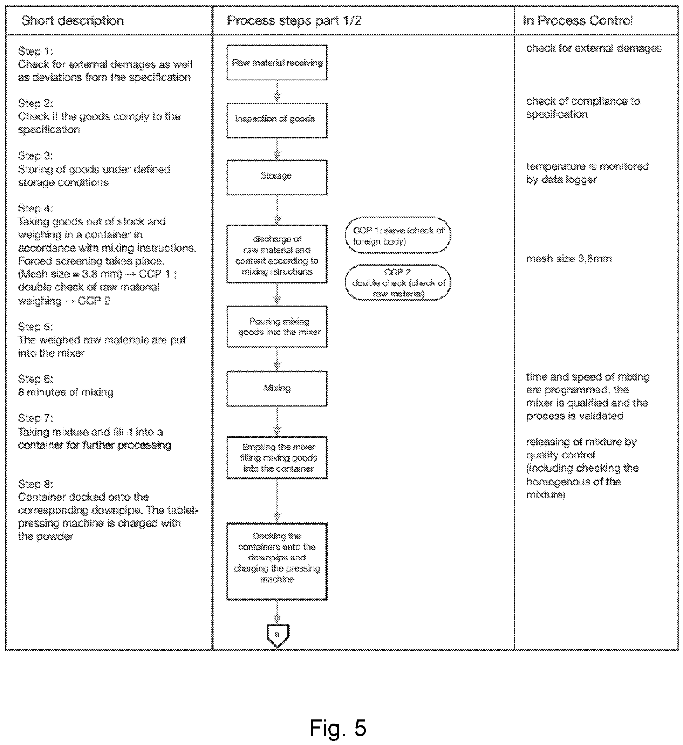

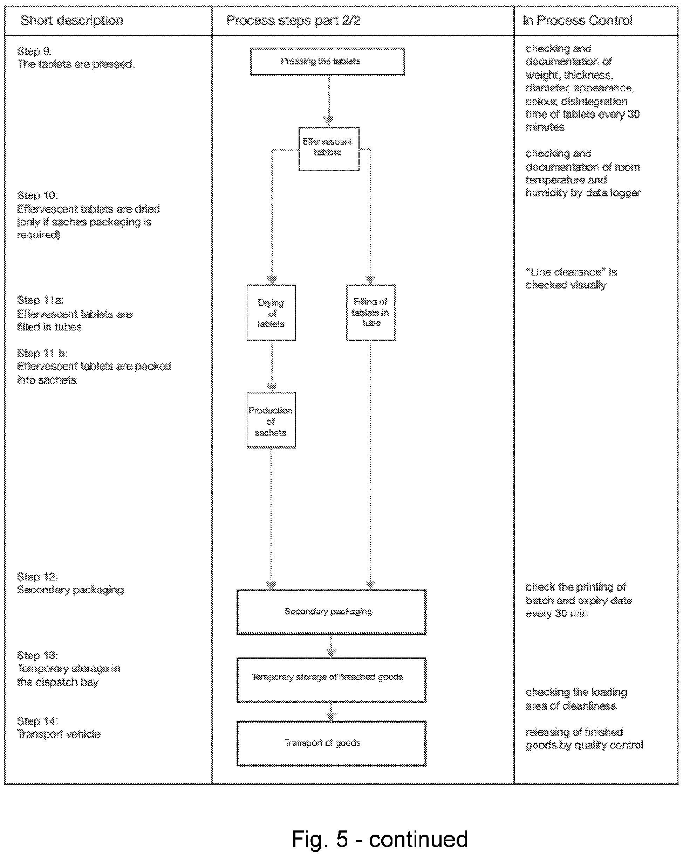

The method described above with reference to FIG. 5 provides that
- the machines used include: Powder Y mixer, Shale Shaker 0.75 mm, Rotary tablet press, Cartooning machine, Technical balances;
- the mixing of the substances occurs as described below: weigh and load the raw materials, pre-sifted with 0,75 mm net, in Y mixer and mix. PEA flakes are previously ground and reduced to a fine powder. The raw materials are introduced in the sequence: purified extract of Propolis, natural extracts and PEA. NAC is introduced last, after the addition of excipients;
- the pharmaceutically acceptable excipients are added to the mixer in the following order: citric acid, sweeteners, flavoring, vitamin B2, sodium bicarbonate.

Transfer the mixture obtained into the rotary tablet press and carry out the compression checking regularly the process control parameters. Load the tablets into the machine hopper. This machine automatically loads the tablets in a container. The container is moved on to the cartooning machine, which works in line with the blistering machine.

The method described in FIG. 6 provides that the different phases of the processing of a solid product with granulation of a hydro-alcoholic extract such as that of Propolis. - the mixing of the substances occurs in the following order: PEA fine powder, natural extracts, NAC. PEA and natural extracts are sifted with a steel vibrating screen with a mesh of 500 pm;
- the addition of the pharmaceutically acceptable excipients occurs in the following order: excipients, anti-caking, flavoring and sweeteners. Mixing is done with a steel Y-mixer. The granulation is done with a double sigma mixer pouring hydro -alcoholic solution with a percentage of ethanol > 80%. The drying, only if required, takes place by placing the dough in a ventilated oven at 40°C for 24 h.

The spraying is done by grinding the dried mixture with the grater Glatt sizing device. Therefore, the invention herein described presents a significant improvement respect to the current commercial products employed for the prevention and/ or the treatment for influenza and/or common colds.

From the above description it is clear, therefore, that the combinations and the production methods as described hereinabove allow to reach the proposed objects. It is similarly evident that, to a person skilled in the art, modifications and variants can be made to the solution described with reference to the attached figures, without departing from the teaching of the present invention and from the scope as defined in the appended claims.

## Claims

1. A combination of food grade substances comprising:
- a first association comprising purified Propolis and PalmitoylEthanolAmide (PEA), and
- a second association comprising PalmitoylEthanolAmide (PEA) and N-Acetyl Cysteine (NAC).

2. A combination of food grade substances comprising:
- purified Propolis,
- PalmitoylEthanolAmide (PEA), and
- N- Acetyl Cysteine (NAC).

3. A combination according to claim 1 or 2 for use as food supplements, functional foods, foods for special medical purposes or medicaments for maintaining the physiological state of health of a user against seasonal illnesses.

4. The combination for use according to claim 3, wherein seasonal illnesses are influenza and/ or common colds.

5. The combination for use according to claim 3 or 4 formulated for buccal, peribuccal, orobuccal or sublingual administration.

6. The combination for use according to claim 5 in the form of orosoluble and/or effervescent powder, tablets, capsules, bars, dragees, pills or granules.

7. A combination according to claim 1, wherein purified Propolis and PalmitoylEthanolAmide (PEA) of the first association are in a ratio by weight ranging between 0.2 and 5 and PalmitoylEthanolAmide (PEA) and N-Acetyl Cysteine (NAC) of the second association are in a ratio by weight ranging between 0.2 and 4.

8. A combination according to claim 2, wherein purified Propolis and PalmitoylEthanolAmide (PEA) and N-Acetyl Cysteine (NAC) are in a ratio by weight ranging between 0.1 and 6.

9. A method to produce food supplements, functional foods, foods for special medical purposes or medicaments for maintaining the physiological state of health of a user against seasonal illnesses comprising the steps of - providing the combination of food grade substances according to claim 1,
- adding pharmaceutically acceptable excipients.

10. A method to produce food supplements, functional foods, foods for special medical purposes or medicaments for maintaining the physiological state of health of a user against seasonal illnesses comprising the steps of
- providing the combination of food grade substances according to claim 2,
- adding pharmaceutically acceptable excipients.

## Patentansprüche

1. Kombination von Substanzen in Lebensmittelqualität, umfassend:
- eine erste Assoziation, die gereinigte Propolis und Palmitoyl-Ethanol-Amid (PEA) umfasst, und
- eine zweite Assoziation, die Palmitoyl-Ethanol-Amid (PEA) und N-Acetyl-Cystein (NAC) umfasst.

2. Kombination von Substanzen in Lebensmittelqualität, umfassend:
- gereinigte Propolis,
- Palmitoyl-Ethanol-Amid (PEA), und
- N-Acetyl-Cystein (NAC).

3. Kombination nach Anspruch 1 oder 2 zur Verwendung als Nahrungsergänzungsmittel, funktionelle Lebensmittel, Lebensmittel für besondere medizinische Zwecke oder Arzneimittel zur Aufrechterhaltung des physiologischen Gesundheitszustandes eines Anwenders gegen saisonale Krankheiten.

4. Kombination zur Verwendung nach Anspruch 3, wobei es sich bei saisonalen Krankheiten um Influenza und/oder Erkältungskrankheiten handelt.

5. Kombination zur Verwendung nach Anspruch 3 oder 4, die zur bukkalen, peribukkalen, orobukkalen oder sublingualen Verabreichung formuliert ist.

6. Kombination zur Verwendung nach Anspruch 5 in Form von schmelzlöslichen und/oder brauselöslichen Pulvern, Tabletten, Kapseln, Riegeln, Dragees, Pillen oder Granulaten.

7. Kombination nach Anspruch 1, wobei gereinigte Propolis und Palmitoyl-Ethanol-Amid (PEA) der ersten Assoziation in einem Gewichtsverhältnis in einem Bereich von 0.2 bis 5 vorliegen, und Palmitoyl-Ethanol-Amid (PEA) und N-Acetyl-Cystein (NAC) der zweiten Assoziation in einem Gewichtsverhältnis in einem Bereich von 0.2 bis 4 vorliegen.

8. Kombination nach Anspruch 2, wobei gereinigte Propolis und Palmitoyl-Ethanol-Amid (PEA) und N-Acetyl-Cystein (NAC) in einem Gewichtsverhältnis in einem Bereich von 0.1 bis 6 vorliegen.

9. Verfahren zur Herstellung von Nahrungsergänzungsmitteln, funktionellen Lebensmitteln, Lebensmitteln für spezielle medizinische Zwecke oder Arzneimitteln zur Aufrechterhaltung des physiologischen Gesundheitszustandes eines Anwenders gegen saisonale Krankheiten, umfassend die Schritte:
- Bereitstellen der Kombination von Substanzen in Lebensmittelqualität gemäß Anspruch 1,
- Zugabe von pharmazeutisch akzeptablen Hilfsstoffen.

10. Verfahren zur Herstellung von Nahrungsergänzungsmitteln, funktionellen Lebensmitteln, Lebensmitteln für besondere medizinische Zwecke oder Arzneimitteln zur Aufrechterhaltung des physiologischen Gesundheitszustandes eines Anwenders gegen saisonale Krankheiten, umfassend die Schritte:
- Bereitstellen der Kombination von Substanzen in Lebensmittelqualität gemäß Anspruch 2,
- Zugabe von pharmazeutisch akzeptablen Hilfsstoffen.

## Revendications

1. Combinaison de substances de qualité alimentaire, comprenant :
- une première association comprenant de la propolis purifiée et du palmitoyléthanolamide (PEA), et
- une seconde association comprenant du palmitoyléthanolamide (PEA) et de la N-acétylcystéine (NAC).

2. Combinaison de substances de qualité alimentaire, comprenant :
- de la propolis purifiée,
- du palmitoyléthanolamide (PEA), et
- de la N-acétylcystéine (NAC).

3. Combinaison selon la revendication 1 ou 2, destinée à être utilisée en tant que compléments alimentaires, aliments fonctionnels, aliments à usage médical spécial ou médicaments pour maintenir l'état de santé physiologique d'un utilisateur à l'encontre de maladies saisonnières.

4. Combinaison pour utilisation selon la revendication 3, dans laquelle les maladies saisonnières sont la grippe et/ou les rhumes.

5. Combinaison pour utilisation selon la revendication 3 ou 4, formulée pour administration buccale, péribuccale, orobuccale ou sublinguale.

6. Combinaison pour utilisation selon la revendication 5, sous forme de poudre, de comprimés, de gélules, de barres, de dragées, de pilules ou de granules orosolubles et/ou effervescents.

7. Combinaison selon la revendication 1, dans laquelle la propolis purifiée et le palmitoyléthanolamide (PEA) de la première combinaison sont à un rapport en poids compris entre 0.2 et 5, et le palmitoyléthanolamide (PEA) et la N-acétylcystéine (NAC) de la seconde combinaison sont à un rapport en poids compris entre 0.2 et 4.

8. Combinaison selon la revendication 2, dans laquelle la propolis purifiée et le palmitoyléthanolamide (PEA) et la N-Acétyl Cystéine (NAC) sont à un rapport en poids compris entre 0.1 et 6.

9. Procédé pour produire des compléments alimentaires, des aliments fonctionnels, des aliments à usage médical spécial ou des médicaments pour maintenir l'état de santé physiologique d'un utilisateur à l'encontre des maladies saisonnières, comprenant les étapes consistant à :
- fournir la combinaison de substances de qualité alimentaire selon la revendication 1,
- ajouter des excipients pharmaceutiquement acceptables.

10. Procédé pour produire des compléments alimentaires, des aliments fonctionnels, des aliments à usage médical spécial ou des médicaments pour maintenir l'état de santé physiologique d'un utilisateur à l'encontre des maladies saisonnières, comprenant les étapes consistant à :
- fournir la combinaison de substances de qualité alimentaire selon la revendication 2,
- ajouter des excipients pharmaceutiquement acceptables.
